(19) Europäisches Patentamt
European Patent Office
Office européen des brevets

(11) EP 1 266 884 A1

(12) **EUROPEAN PATENT APPLICATION**
published in accordance with Art. 158(3) EPC

(43) Date of publication:
**18.12.2002 Bulletin 2002/51**

(21) Application number: **01914179.5**

(22) Date of filing: **21.03.2001**

(51) Int Cl.[7]: **C07C 211/53**

(86) International application number:
**PCT/JP01/02204**

(87) International publication number:
**WO 01/070667 (27.09.2001 Gazette 2001/39)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **22.03.2000 JP 2000080081**

(71) Applicant: **BF Research Institute, Inc.**
**Osaka-shi, Osaka 532-8686 (JP)**

(72) Inventors:
- **KUDO, Yukitsuka**
  **1-chome Suita-shi, Osaka 565-0842 (JP)**
- **SUZUKI, Masako**
  **Suita-shi, Osaka 564-0022 (JP)**
- **SUEMOTO, Takahiro**
  **Minoh-shi, Osaka 562-0031 (JP)**
- **SHIMAZU, Hiroshi**
  **Settsu-shi, Osaka 566-0024 (JP)**

(74) Representative: **Vossius, Volker, Dr. et al**
**Patent- und Rechtsanwaltskanzlei**
**Geibelstrasse 6**
**81679 München (DE)**

(54) **IMAGE DIAGNOSIS PROBE BASED ON SUBSTITUTED AZOBENZENE OR ANALOGUE THEREOF FOR DISEASE ATTRIBUTABLE TO AMYLOID ACCUMULATION AND COMPOSITION FOR IMAGE DIAGNOSIS CONTAINING THE SAME**

(57) A compound represented by the formula (I):

$$(R_1)_m—A—X\text{-----}Y—B—(R_2)_n \qquad (I)$$

or a salt or a solvate thereof; and a composition and a kit both containing any of these. In said formula I, X represents CH, Sulfur, or nitrogen; Y represents CH, sulfur or nitrogen, or absent; ----- means a single bond or a double bond; A and B each independently represents a benzene ring or six-membered heterocyclic containing one or two atoms of nitrogen, oxygen, or sulfur; and $R_1$ and $R_2$ represent one to seven substituents of ring A and ring B, respectively.

EP 1 266 884 A1

## Description

Imaging diagnostic probe for diseases in which amyloid is accumulated using substituted azobenzene and similar compounds thereto, and a composition for imaging diagnosis comprising them.

### Field of the Invention

The present invention relates to an imaging diagnostic probe for diseases in which amyloid is accumulated, more particularly, a probe labeled with a positron-emitting nuclide, and an composition for imaging diagnosis, comprising the prove.

### Prior art

Diseases in which amyloid is accumulated include various diseases characterized by deposition of an insoluble fibrillar protein (amyloid) on various organs or tissues in the body, such as Alzheimer's disease and Down's syndrome. Among them, Alzheimer's disease (AD) is currently one of diseases which are most difficult to treat, and precise early diagnosis is desired.

Alzheimer's disease is characterized by progressive dementia occurring mainly in presenility to senium. Pathologically, Alzheimer's disease is characterized in atrophy of brain, remarkable neurodegeneration and loss of neuronal cells, appearance of neurofibrillary tangle and senile plaque. It is known that the greatest risk factor for dementia, a representative of which is Alzheimer's disease, is aging. Therefore, increase in the number of patients with increase in an elderly population is remarkable, particularly in Japan, United States of America and European countries which have grown into an aging society, and in these countries the medical cost therefor has put the medical system in difficult situation.

In Japan, the number of Alzheimer's disease patients is presumed to be one million, and it is considered that the number of patients will be surely increasing with aging of a population from now on. Since the cost for an Alzheimer's patient is thought to exceed 2.5 million yen per patient per year, including the nursing cost, Japan has already spent the social and economical cost exceeding 2.5 trillion-yen. Treatment of Alzheimer's disease before obvious appearance of a dementia symptom or at an early stage brings a great medical and economical effect, which is worldwide common sense now. However, under the current circumstances, it is extremely difficult to precisely diagnose Alzheimer's disease at these stages.

At present, there are various methods for diagnosing Alzheimer's diseases. In Japan, a method of quantitatively evaluating decrease in the cognition function of individuals suspected of Alzheimer's disease is general, for example Hasegawa's method, ADAS, MMSE and the like and, rarely, an imaging diagnostic method (MRI, CT etc.) is used supplementarily. However, these diagnosing methods are insufficient for identifying diseases and, thus, for definite diagnosis, biopsy of a brain before death, and pathological and histological examination of a brain are necessary after death. Like this, although a method for diagnosing Alzheimer' s disease has been intensively studied, progression is hardly recognized. As a result of many studies, it has been found that neuronal degeneration characteristic of Alzheimer's disease initiates considerably before first appearance of the clinical symptom (about 40 years in a longer case). It is known that, in the same disease, when a family or a clinician surrounding a patient recognizes the first clinical symptom, the pathological feature in a brain of the patient has progressed to the irreversible stage. Taking into consideration the aforementioned progress property of the condition and a drastic increase in the number of patients, precise diagnosis of Alzheimer's disease at an early stage is greatly important and significant.

The pathological feature for Alzheimer's disease represents two main characteristics. That is, senile plaque and neurofibrillary tangle. The main component of the former is the amyloid β protein having β sheet structure, and the main component of the latter is excessively phosphorylated Tau protein. Definite diagnosis of Alzheimer's disease relies on appearance of these pathological characteristics in a patient' s brain. Regarding the significance of amyloid β protein in the development mechanism for Alzheimer's disease, the followings have been known (see Katsuhiko Yanagihara, Yasuo Ihara: Progress in Neural Study, vol.41, p.70-79, 1997, Mikio Shoji: Dementia Japan, vol.11, p. 43-50, 1997, Akira Tamaoka: Dementia Japan, vol.11, p.51-57, 1997).

1. Diffuse deposition of the amyloid β protein (Aβ) is an earliest neural pathological change in a brain of Alzheimer's disease.
2. There is familial Alzheimer's disease having a point mutation in a gene of an Aβ precursor, amyloid precursor protein (APP).
3. Abnormal production of Aβ is recognized in a cultured cell in which the gene described in above 2 has been introduced.
4. Abnormal production of Aβ is also recognized in preseniline gene abnormality which occupies a majority of

familial Alzheimer's disease.

5. In a brain of Down's syndrome having trisomy on a twenty-first chromosome in which a gene encoding APP is present, the similar neuropathologfical change to that of a brain of Alzheimer's disease appears at an early stage.

As described above, the amyloid β protein is characteristic in diseases in which amyloid is accumulated, including Alzheimer's disease, and has the close relationship with these diseases. Therefore, detection using as a marker the amyloid β protein having β sheet structure in the body, in particular, in a brain, becomes one of important diagnosing methods for diseases in which amyloid is accumulated, in particular Alzheimer's disease.

To diagnose diseases in which amyloid is accumulated, including Alzheimer's disease, search for substances which specifically bind to the amyloid β protein in the body, in particular in a brain, and stain the protein, has been previously carried out. As such the substance, there have been reported only Congo Red (Puchtler et al., Journal of Histochemistry and Cytochemistry, vol.10, p.35, 1962), Thioflavin S (Puchtler et al.,Jouranl of Histochemistry and Cytochemistry, vol.77, p.431, 1983), Thioflavin T (LeVine, Protein Science, vol.2, p.404-410, 1993) and Chrysamine G and derivatives thereof (International Patent Application PCT/US96/05918 and PCT/US98/07889). However compounds belonging to a group other than the aforementioned group has not been reported. Reported compounds have not a few problems from a viewpoint of the binding specificity to the amyloid β protein, the blood-brain barrier permeability, the solubility, the toxicity and the like. Therefore, at present, these compounds have not been put into practice yet in diagnosis of diseases in which amyloid is accumulated.

In view of the aforementioned circumstances, the present invention provides compounds belonging to a different group from that of previous compounds, which have the high binding specificity to the β amyloid protein and blood-brain barrier permeability, and can be used as probes for imaging diagnosis of diseases in which amyloid is accumulated. Also, the present invention relates to substances labeled for using as probes for imaging diagnosis of diseases in which amyloid is accumulated, and relates to an imaging diagnostic composition comprising such a probe.

## Summary of the Invention

In order to solve the aforementioned problems, the present inventors studied intensively and found that compounds represented by the formula I or salts or a solvates thereof have very high binding specificity to the β amyloid protein, blood-brain barrier permeability, and finally completed the present invention.

That is, the present invention relates to:

(1) a compound used as a probe for imaging diagnosis of diseases in which amyloid is accumulated, represented by the formula I:

$$(R_1)_m—A—X\underline{----}Y—B—(R_2)_n \qquad (I)$$

[wherein X represents CH, N or S;

Y represents CH, N or S; or absent;

<u>----</u> means a single bond or a double bond;

A and B each represents independently carbocyclic ring selected from benzene ring, naphthalene ring, anthracene ring and phenanthrene ring; or represents said carbocyclic ring containing one to three heteroatoms selected from N, O and S;

$R_1$ represents a substituent for ring A, and each $R_1$ represents independently hydrogen, halogen, hydroxy, =O, alkyl having 1-4 carbons, -0-alkyl having 1-4 carbons, nitro, -N=S, amino, -O- alkyl having 1-4 carbons, mono-substituted amino with an alkyl having 1-4 carbons, di-substituted amino with alkyls having 1-4 carbons, $-NHCONH_2$, $-NHCOCH_2$-halogen, carboxyl, sulfonic acid group, $-SO_2$-halogen, $-SO_2$-OH, or

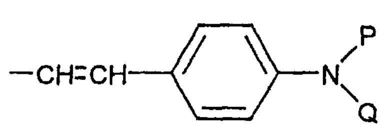

(wherein, P and Q each independently represents alkyl having 1-4 carbons);

$R_2$ represents a substituent for ring B; each $R_2$ represents independently hydrogen, halogen, hydroxy, =O

nitro, -N=S, amino, mono-substituted amino with an alkyl having 1-4 carbons, di-substituted amino with alkyls having 1-4 carbons, carboxyl, sulfonic acid group, or

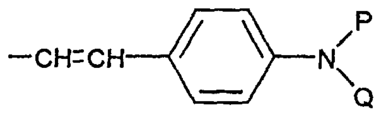

(wherein, P and Q independently are alkyl having 1-4 carbons) ;
n means any number of 1 to 7;
m means any number of 1 to 7;
provided -B- $(R_2)_n$ represents

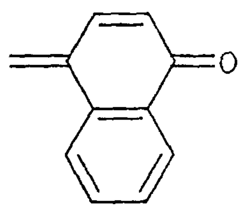

when Y means absent], or a salt or a solvate thereof,
(2) a compound described in (1) selected from the group consisting of:

N-039 (N-[4-(2-{6-[4-(dimethylamino)styryl]-1-methylpiridinium-2-yl}vinyl)phenyl]-N-methylmethanamine),
BF-064 (N-[4-(2-{6-[4-(dimethylamino)styryl]-1-methylpiridinium-2-yl}vinyl)phenyl]-N-ethylmethanamine) or
SA-215 (4-(2-pyridylazo)-N,N-dimethylaniline),

or a salt or a solvate thereof,
(3) the compound or a salt or a solvate thereof described in (1) or (2), which is labeled,
(4) the compound or a salt or a solvated thereof described in (3), wherein the label is a radioactive nuclide,
(5) the compound or a slat or a solvate thereof described in (4), wherein any of substituents $R_1$ to $R_2$ are labeled with a radioactive nuclide,
(6) the compound or a salt or a solvate thereof described in (4), wherein hydrogen on the ring is substituted with a radioactive nuclide,
(7) the compound or a salt or a solvate thereof described in any one of (4) to (6), wherein the label is a γ- ray-radiating nuclide,
(8) the compound or a salt or a solvate thereof described in (7), wherein the γ-ray-radiating nuclide is selected from the group consisting of $^{99m}Tc$, $^{111}In$, $^{67}Ga$, $^{201}Tl$, $^{123}I$ and $^{133}Xe$,
(9) the compound or a salt or a solvate thereof described in (7), wherein the γ-ray-radiating nuclide is selected from the group consisting of $^{99m}Tc$ and $^{123}I$,
(10) the compound or a salt or a solvate thereof described in any one of (4) to (6), wherein the label is a positron-radiating nuclide,
(11) the compound or a salt or a solvate thereof described in (10), wherein the positron-emitting nuclide is selected from the group consisting of $^{11}C$, $^{13}N$, $^{15}O$ and $^{18}F$,
(12) the compound or a salt or a solvate thereof described in (10), wherein a positron-emitting nuclide is $^{18}F$,
(13) a composition for imaging-diagnosis of diseases in which amyloid is accumulated, which comprises the compound or a pharmaceutically acceptable salt or a solvate thereof described in any one of the above (1) to (12) and a pharmaceutically acceptable carrier,
(14) the composition described in (13), which contains a compound selected from the group consisting of N-039, BF-064 and SA-205 labeled with $^{99m}Tc$ or $^{123}I$ or a pharmaceutically acceptable salt or a solvate thereof,
(15) the composition described in (13), which contains a compound selected from the group consisting of N-039, BF-064 and SA-205 labeled with $^{18}F$ or a pharmaceutically acceptable salt or solvate thereof,
(16) a kit for imaging-diagnosis of diseases in which amyloid is accumulated, which comprises as an essential component the compound or a pharmaceutically acceptable salt or a solvate thereof described in any one of above (1) to (12),

(17) the kit described in (16), which comprises as an essential component a compound selected from the group consisting of N-039, BF-064 and SA-205 labeled with $^{99m}Tc$ or $^{123}I$ or a pharmaceutically acceptable salt or a solvate thereof,

(18) the kit described in (16), which comprises as an essential component a compound selected from the group consisting of N-039, BF-064 and SA-205 labeled with $^{18}F$ or a pharmaceutically acceptable salt or a solvate thereof, and

(19) a use of the compound or a salt or a solvate thereof described in any one of the above (1) to (12), for preparing a composition or a kit for imaging-diagnosis of diseases in which amyloid is accumulated.

Detailed Description of the Invention

A substance used as a probe for imaging diagnosis of diseases in which amyloid is accumulated of the present invention is a compound represented by the general formula I or a salt or a solvate thereof.

Each substituent of the compound of the formula I will be explained below. "Alkyl having a carbon number of 1 to 4" as used herein includes methyl, ethyl, propyl, butyl and a structural isomer thereof.

X represents CH, Nor S. Y represents CH, Nor S; or absent. ----- represents single bond or double bond. Examples of X ---- Y part include N=N, CH=N, N=CH, S-S.

A preferable ring for ring A and ring B is benzene ring.

Among benzene ring, naphthalene ring anthracene ring and phenanthrene ring, containing one to three heteroatoms selected from N, O or S, benzene ring and pyridine ring are preferable.

$R_1$ and $R_2$ are one to at most seven substituents for ring A and ring B, respectively, positions of which are any possible positions of the ring. Ortho or para substituents are preferable for $R_1$ and $R_2$. In addition, when ring A or ring B is heterocycle , substituent $R_1$ or $R_2$ can exist on the heteroatom (N, S or O).

When $R_1$ or $R_2$ is alkyl having one to four carbons, preferably it is methyl, ethyl, or propyl.

When $R_1$ or $R_2$ is halogen, preferably it is fluorine, chlorine or iodine.

$R_1$ or $R_2$ which is mono-substituted amino with an alkyl having one to four carbons includes, but not limited to, methylamino and ethylamino groups. $R_1$ or $R_2$ which is di-substituted amino with alkyls having one to four carbons includes, but not limited to, dimethylamino and diethylamino groups. When $R_1$ or $R_2$ is di-substituted amino with alkyl having one to four carbons, it can be a form of onium ion on the nitrogen, and an onium salt can be formed with an anoin as described below. Further, when $R_1$ or $R_2$ is carboxyl or sulfonic acid group, a salt can be formed thereon (such a salt is described below). In addition, when the compound of formula I contains a hydoxy group (for example $R_1$ or $R_2$ is hydroxy group), the compound of formula I can form a keto-enol tautomer there. Such an isomer is included within the present invention. Moreover, hydrogen of hydroxy group can be substituted by a metal (for example sodium, potassium).

When Y is absent, B-$(R_2)_n$ is

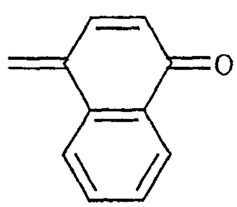

examples of this type of the compound of formula I are indophenol blue and 5-(4-dimethylaminobenzylidene)rhodamine (SA-447) as shown in Table I.

Particularly preferred compounds of the present invention are described below.

A compound is preferred in which X=Y is CH=CH, rings A and ring B are pyridine rings, $R_1$ and $R_2$ are

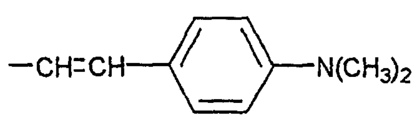

Typical example of such a compound is N-039, i.e. N-[4-(2-{6-[4-(dimethylamino)styryl]-1-methylpiridinium-2-yl}vinyl)phenyl]-N-methylmethanamine (see Table I).

A compound is preferred in which X=Y is CH=CH, rings A and ring B are pyridine rings, $R_1$ and $R_2$ are

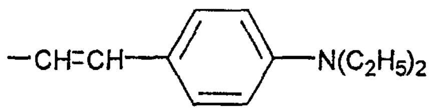

Typical example of such a compound is BF-064, i.e. N-[4-(2-{6-[4-(dimethylamino)styryl]-1-methylpiridinium-2-yl}vinyl)phenyl]-N-ethylmethanamine (see Table I).

A compound is preferred in which X=Y is N=N, ring A is benzene ring, ring B is pyridine ring, $R_1$ is $N(CH_3)_2$ (m=1), $R_2$ is H. Typical example of such a compound is SA-215, i.e. 4-(2-pyridilazo)-N,N-dimethylaniline (see Table I).

Typical examples of the compounds of formula I are shown in Table I below. The compound of formula I can form a salt with various ions, and such a salt is included within the present invention. A salt may be formed with any functional group in the compound of the formula I. For example, when a carboxyl group or a sulfonic acid group is present in the compound as described above, a salt may be formed between this and a metal. Examples of such the salt include salts with an alkali metal such as lithium, sodium and potassium, and salts with an alkaline earth metal such as magnesium, calcium and barium. In addition, as described above, the compound of formula I can form an onium salt. Examples of the anion forming an onium salt with the compound of formula I include, but not limited to, a halide ion, an organic acid ion, sulfonic acid ion. Such an onium salt is also included within the present invention. Further, complexes formed by the compound of the formula I and a metal salt (e.g. complexes formed with a metal salt such as magnesium chloride and iron chloride) are included in salts of the compound of the formula I in the present specification. When the compound of the present invention is used in a composition or a kit, the salt is preferably a pharmaceutically acceptable salt. Examples of the pharmaceutically acceptable salt of the compound of the formula I include forms of an onium salt with a halide ion such as chlorine, bromine and iodine, as well as salts with a metal such as sodium, potassium and calcium, and complexes formed with a metal salt such as iron chloride and cobalt chloride. These salts of the aforementioned compound of the formula I are included in the present invention. In addition, solvates of the compound of the formula I are included in the present invention. Examples of the solvate include hydrate, methanolate, ethanolate. aqmmoniate and the like. When used in the present composition or kit, a pharmaceutically acceptable solvate is also preferable, and examples of the pharmaceutically acceptable solvate include hydrate, ethanolate and the like. "compound(s) of the present invention" as used herein includes a compound of the formula I, and a salt and a solvate thereof.

In the present invention, compounds of the formula I or salts or solvates thereof which specifically bind to amyloid, particularly β amyloid protein in vivo in the body in diseases in which amyloid is accumulated, are used as a probe for imaging diagnosis of diseases in which amyloid is accumulated. "Diseases in which amyloid is accumulated" as used herein is characterized in deposition of the amyloid protein, particularly the β amyloid protein in the body as described above, and that disease refers to those which can be diagnosed using deposition of β amyloid protein as a marker, including Alzheimer's disease, Down's syndrome and the like.

In diagnosis of diseases in which amyloid is accumulated, generally the labeled compound of the present invention is used as a probe. Example of the label include a fluorescent substance, an affinity substance, an enzyme substrate, a radioactive nuclide and the like. A probe labeled with a radioactive nuclide is usually used for imaging-diagnosing diseases in which amyloid is accumulated. The compound of the present invention can be labeled with various radioactive nuclides by the methods well known in the art. For example, $^{3}H$, $^{14}C$, $^{35}S$, $^{13}I$ and the like are radioactive nuclides which have previously been used, and utilized in vitro in many cases. The general requirements for a probe for the imaging diagnosis and a detecting means therefor are: feasibility of in vivo diagnosis, a small damage to a patient (in particular, being non-invasive), a high sensitivity of detection, a suitable length of half time (a suitable length of time for preparing a labeled probe, and for diagnosis) etc. Then, recently, a positron emission tomography (PET) utilizing γ-ray with the high detection sensibility and substance permeating property has been used, or a single photon computer tomography (SPECT) by γ-ray-releasing nuclides have been used. Among them, since PET detects two γ-rays radiated from a positron-emitting nuclide in forward and reverse directions, using a pair of detectors by a simultaneous counting method, and the information obtained is excellent in a resolving power and a quantitative property, PET is a preferable method. For SPECT, the compound of the present invention can be labeled with a γ-ray-releasing nuclide such as $^{99m}Tc$, $^{111}In$, $^{67}Ga$, $^{201}Tl$, $^{123}I$, $^{133}Xe$ and the like. $^{99m}Tc$ and $^{123}I$ are frequently used in SPEC. For PET, the compound of the present invention can be labeled with a positive electron-releasing nuclide such as $^{11}C$, $^{13}N$, $^{15}O$, $^{18}F$, Cu, $^{68}Ga$, $^{76}Br$ and the like. Among the positron-emitting nuclides, $^{11}C$, $^{13}N$, $^{15}O$ and $^{18}F$ are preferable from a viewpoint of a suitable half life and easy labeling, and $^{18}F$ is particularly preferable. A position in the compound of the present invention to be labeled with a radiation-releasing nuclide such as a positive electron-releasing nuclide, a γ-ray-radiating nuclide and the like may be any position of the compounds of the formula I. For example,

substituent $R_1$ or $R_2$ of the compound of formula I can be labeled with a radioactive nuclide such as a positron-emitting nuclide, a γ-ray radiating nuclide, etc. Or, a hydrogen on the ring of the compound of formula I can be substituted by a radioactive nuclide such as a positron-emitting nuclide, a γ-ray radiating nuclide, etc. Such a labelled compound is also included within the present invention. For example, when the compound of present invention is labelled with $^{18}F$, substituent $R_1$ or $R_2$ can be labelled with $^{18}F$, or hydrogen on the ring of the compound of formula I can be substituted by $^{18}F$. For example, hydrogen contained in substituent $R_1$ or $R_2$ on ring A or ring B can be substituted by $^{18}F$. A compounds of the present invention suitable for labeling with a radioactive nuclide such as $^{18}F$ includes, but not limited to, N-039, BF-064 and SA-215 as described above. Generally, these nuclides are produced by an apparatus called a cyclotron or a generator. A person skilled in the art can select a producing method and an apparatus depending on a nuclide to be produced. The compound of the present invention can be labeled using the thus produced nuclide.

The process for preparing a labeled compound labeled with these radioactive nuclides is well known in the art. As representative methods, there are a chemical synthesizing method, an isotope exchanging method and a biosynthesis method. The chemical synthesizing method has previously been used widely, and is not substantially different from a normal chemical synthesizing method except that a radioactive starting material is used. By this method, various nuclides have been introduced in compounds. The isotope exchanging method is a method in which $^{3}H$, $^{35}S$, $^{125}I$ and the like in a compound having a simple structure are transferred in a compound having a complicated structure, and a compound having a complicated structure labeled with these nuclides is obtained. A biosynthesis method is a method in which a compound labeled with $^{14}C$, $^{35}S$ or the like is given to a cell such as a microorganism, and a metabolite with these nuclides introduced is obtained.

Regarding a position to be labeled, a label can be introduced into a desired position by designing a synthesis scheme depending on a purpose, like a normal synthesis. Such the design is well known to a person skilled in the art.

In addition, for example, when a positron-emitting nuclide such as $^{11}C$, $^{13}N$, $^{15}O$, $^{18}F$, etc having a relatively short half life is used, a desired nuclide is obtained from a (super) miniature cyclotron arranged in facilities such as hospital and the like, and a desired compound is labeled at a desired position by the aforementioned method, which can be immediately used for diagnosis, examination, treatment and the like.

By these methods known to a person skilled in the art, labeling can be performed by introducing a desired nuclide into a desired position of the compound of the present invention.

The labeled compound of the present invention may be administered to a subject locally or systemically. Examples of route of administration include intradermal, intraperiteneal, intravenous, intraarterial, or intraspinal injections or infusions, and route of administration can be selected depending on factors such as kind of disease, a nuclide to be used, a compound to be used, the condition of a subject, an examination site, etc. The present probe is administered and, after a sufficient time for binding to the β amyloid protein and disintegration, an examination site can be examined by means such as PET, SPECT, etc. These means can be appropriately selected depending on factors such as a kind of disease, a nuclide to be used, a compound to be used, the condition of a subject, an examination site, etc.

A dose of the compound of the present invention labeled with a radioactive nuclide varies depending on a kind of disease, a nuclide to be used, a compound to be used, an age of a subject, the physical condition, sex, a degree of disease, an examination site and the like. In particular, a sufficient attention must be paid to a dose exposed to a subject. For example, a radioactivity amount of the compound of the present invention labeled with a positive electron-releasing nuclide such as $^{11}C$, $^{13}N$, $^{15}O$ and $^{18}F$ is usually in a range of 3.7 megabecquerels to 3.7 gigabecquerels, preferably 18 megabecquerels to 740 megabecquerels.

Also, the present invention provides a composition for imaging-diagnosis of diseases in which amyloid is accumulated, which comprises the compound of the present invention. The present composition comprises the compound of the present invention and a pharmaceutically acceptable carrier. It is preferable that the compound of the present invention in the composition is labeled. There are various labeling methods as described above, but it is desirable that the compound of the present invention is labeled with a radioactive nuclide (in particular, a positron-emitting nuclide such as $^{11}C$, $^{13}N$, $^{15}O$ and $^{18}F$) for use in imaging diagnosis in vivo. It is preferable that the form of the present composition is an injectable or infusionable form a viewpoint of the purpose. Therefore, the pharmaceutically acceptable carrier is preferably liquid, and includes, but not limited to water-soluble solvents such as potassium phosphate buffer, physiological saline, Rilger's solution, distilled water and the like, and non-water-soluble solvents such as polyethylene glycol, vegetable fat or oil, ethanol, glycerin, dimethyl sulfoxide, propylene glycol. A ratio of a the carrier and the compound of the present invention to be incorporated can be appropriately selected depending on an application site, a detecting means and the like, and is usually a ratio of 100 thousands vs. 1 to 2 vs. 1, preferably a ratio of 10 thousands vs. 1 to 10 vs. 1. In addition, the present composition may further contain the known antibacterial agent (for example, antibiotic etc.), local anesthetic (for example, procaine hydrochloride, dibucaine hydrochloride etc.), buffer (for example, Tris-HCl buffer, HEPES buffer etc.), osmotic pressure adjusting agent (for example, glucose, sorbitol, sodium chloride etc.) and the like.

Further, the present invention provides a kit for imaging diagnosis of diseases in which amyloid is accumulated, which contains the compound of the present invention as an essential component. Usually, a kit is a combination of

respective containers in which respective components such as the compound of the present invention, a solvent for dissolving the same, a buffer, an osmotic pressure adjusting agent, an antibacterial agent, a local anesthetic, etc are placed therein individually, or some of respective components are placed therein together. The compound of the present invention may be unlabeled or may be labeled. When unlabeled, the compound of the present invention may be labeled prior to use by the normal method as explained above, In addition, the compound of the present invention maybe provided as a solid such as a lyophilized powder or the like, or may be provided by dissolving in a suitable solvent. The solvent may be the same as the carrier used in the present composition as described above. In addition, respective components such as a buffer, an osmotic pressure adjusting agent, an antibacterial agent, a local anesthetic and the like may be the same as those used in the composition of the present invention as described above. Various containers may be appropriately selected, and form and shape for operations of introducing a label into the compound of the present invention may be adopted, a material having light blocking property may be used depending on the nature of a compound, or the container may have a form such as a vial, an injector or the like for convenient administration to the patient. In addition, the kit may appropriately contain equipments necessary for diagnosis, for example, an injector and an infusion set, or equipments used in the PET apparatus. Usually, an instruction is attached to the kit.

Further, since the compound of the present invention specifically binds to the amyloid β protein, the compound of the present invention may be unlabeled or may be labeled for use in detection, quantification or the like of the amyloid β protein in vitro. For example, the compound of the present invention may be used in staining of the amyloid β protein for a microscope specimen, a colorimetric quantification of amyloid β protein in a sample or quantification of the amyloid β protein using a scintillation counter.

Then, a method of screening the compound of the present invention will be explained.

(1) Method of quantifying the amyloid β protein having β sheet structure -- method of measuring of recognition of β structure in a test compound.

Some methods of quantifying the amyloid β protein having β sheet structure have been already reported, and these methods were modified for the test in the present invention, with reference to the method by LeVine et al. (Protein Science, vol.2, p.404-410, 1993, and woods et al. (Journal of Molecular Biology, vol.256, p.870-877, 1996). That is, the amyloid β protein (purchased from Peptide Laboratory) was dissolved in a potassium phosphate buffer (pH7.4) and allowed to stand at 37°C for 4 days. A test compound dissolved in the same buffer (final concentration 1μM) was dispensed into a 96-well microplate at an each volume of 50 microliters and, thereafter, the amyloid β protein solution which had been allowed to stand for 4 days was added at an each volume of 50 microliters. Then, each 100 microliters of thioflavin T (which emits fluorescence depending on an extent of β structure of the amyloid β protein) dissolved in a glycine-NaOH buffer (pH 8.5) was added (final concentration 3μM), and the fluorescence was immediately measured with a fluorescent microplate reader (manufactured by Molecular Device, Model fmax) at an excitation wavelength of 442 nm and a measurement wavelength of 485 nm. When the fluorescence of thioflavin alone was regarded as A, the fluorescence under the coexistence of the amyloid β protein and thioflavin T was regarded as B, and the fluorescence under the coexistence of the amyloid β protein, thioflavin T and a test compound was regarded as C, the recognition of β structure for the test compound was calculated by the following formula (unless otherwise indicated, the concentration of a test compound was 1μM) :

$$\beta \text{ structure recognition for test compound } (\%) = \{(B-C)/(B-A)\} \times 100$$

As the β structure recognition becomes larger, it can be said that the test compound has the higher binding specificity to the amyloid β protein.

(2)Method of measuring insulin β structure recognition.

It is known that proteins having β sheet structure such as insulin, amylin, etc are present in the human body in addition to the amyloid β protein (Burket et al., Biochemistry, vol. 11, p. 2435-2439, 1972; Ashburn et al, Chemistry and Biology, vol. 3, p. 351-358,1996). It is desirable that a probe for diagnosing diseases in which amyloid is accumulated, including Alzheimer's disease by recognizing the amyloid protein, has the higher affinity with the amyloid β protein and has the lower affinity with other proteins having β sheet structure. Then, the affinity of each compound to insulin having β structure was measured.

A method of quantifying β sheet structure of insulin in vitro was slightly modified to be used in the test of the present invention, with reference to the report by Klunk et al. (Journal of Histochemistry and Cytochemistry, vol. 37, p.1237-1281, 1989). That is, insulin (purchased from Sigma) was dissolved in deionized water, adjusted to pH2 with hydrochloric acid, heated at 92°C for 10 minutes, and cooled in a dry ice/ethanol cooling bath. Thereafter, heating at 92°C for 3 minutes and cooling in a dry ice/ethanol cooling bath were repeated 7 or 8 times. This insulin solution was further diluted to 0.1 mg/ml with a phosphate buffer (pH 7.4) , and dispensed in a 96-well microplate at each volume of 50 microliters. A test compound dissolved in deionized water was dispensed at a volume of 50

microliters, and thiofravin T (emitting fluorescence depending on an extent of β structure) dissolved in a glycine-NaOH buffer (pH 8.5) was dispensed at each volume of 100 microliters, and then the fluorescent extent was measured with a fluorescent microplate reader (manufactured by Molecular Device, Model fmax) at an excitation wavelength of 442 nm and a measurement wavelength of 485 nm. When the fluorescence of thioflavin T alone was regarded as D, the fluorescence under the coexistence of insulin and thioflavin T was regarded as E, and the fluorescence under the coexistence of insulin, thioflavin T and a test compound was regarded as F, the insulin β structure recognition for the test compound was calculated by the following formula:

$$\text{Insulin } \beta \text{ structure recognition for test compound (\%)} = \{(E-F)/(E-D)\} \times 100$$

based on this value, by using the BSAS (Biological Statistical Analysis System) statistic program the 50% effective concentration ($EC_{50}$) for recognizing an insulin β sheet structure for the test compound was obtained.

(3) Method of measuring distribution coefficient for test compound. It is known that a distribution coefficient of a compound between water and lipid is an index of the blood-brain barrier permeability of the compound (Begley, Journal of pharmacy and Pharmacology, vol. 48, p.136-146, 1996, and Buchwald and Boder, Current Medicinal Chemistry, vol.5, p.353-380, 1988).

Then, a distribution coefficient between water/1-octanol was measured and used as an index of the blood-brain barrier permeability.

As an oily phase, 1-octanol was used and, as an aqueous phase, a phosphate buffer (pH 7.3) or ultra-pure water was used. A suitable amount of a test compound was dissolved in an oily phase or an aqueous phase, and both phases were placed in the same test tube, and shaken vigorously at room temperature for 30 minutes. After allowing to stand at room temperature over 1 hour, centrifugation at 2000 rpm for 10 minutes was carried out, followed by further standing at room temperature for 1 hour. The aqueous phase and the oily phase were sampled, respectively, and transferred to a 96-well microplate. By using a microplate reader (manufactured by Molecular Device, Model Spectramax 250), the absorbance was measured at a maximum absorbing wavelength for each test compound, and the concentration of the test compound was calculated from a calibration curve previously obtained. A distribution coefficient was calculated by the following formula.

Distribution coefficient for test compound

= (concentration of test compound in oily phase) /

(concentration of test compound in aqueous phase)

As the distribution coefficient becomes larger, it can be said that the blood-brain barrier permeability for the test compound is higher.

(4) Calculation of usefulness coefficient

When a product of the β structure recognition (specificity of binding of a test compound to the amyloid β protein) and the distribution coefficient (blood-brain barrier permeability for a test compound) is defined as a usefulness coefficient, it is thought that the usefulness coefficient is an index of an amount of a test compound bound to the amyloid β protein in a brain after the test compound has passed through the blood-brain barrier upon actual administration of the test compound to human being.

The usefulness coefficient for the test compound was calculated by the following formula:

Usefulness coefficient for test compound

= (β structure recognition for test compound) x

(distribution for test compound)

As the usefulness coefficient becomes larger, it can be said that the compound is suitable for a probe for diagnosing diseases in which amyloid is accumulated.

(5) Acute toxicity test

The acute toxicity for the compound of the present invention was studied by intravenous administration to a mouse . Five weeks aged Crj:CD1 male mice were used (four animals/group) (average weight in each group was

30 to 31g). Each compound was dissolved in a physiological saline (Otsukaseiyaku K.K.) and a single intravenous administration at 10mg/ml or 100 mg/kg was performed via tail vein, followed by observation until 7 days after administration.

Examples

The present invention is specifically explained by way of Examples, but the Examples do not limit the present invention.

β structure recognition was measured for compounds shown in Table I below as typical compounds of the present invention. For compound N-039, the distribution coefficient was measured by the method of above (4), and the calculated usefulness coefficient. Unless particularly described, these compounds are the highest grade commercially available or synthesized by order (purity>98%). The results are shown in Table I. Values in Table I are averages obtained by duplicated experiments. As a control, the results measured under the same conditions regarding the compounds which have previously been known to bind to the amyloid β protein (Congo red, Chrysamine G and its disodium, Compound X34 (1,4-bis[2-(3-carboxy-4-hydroxyphenyl)eten-yl]-benzene, synthesized by Tanabe R & D Service ) and its disodium salt (synthesized by Tanabe R & D, Service)) are also shown in Table I. The structural formulas in Table I show one of examples of possible forms of the compound listed. Blanks in Table I represent "not measured".

Table I

| Compound | β Structure recognition (%) | Distribution coefficient | Usefulness coefficient | Structure |
|---|---|---|---|---|
| N-039 | 97.7 | 35 | 3420 | 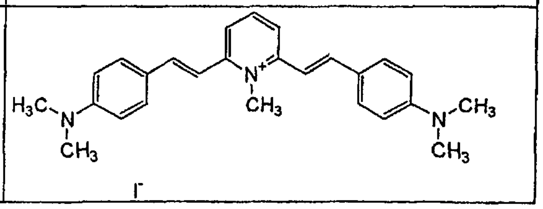 |

| | | | | |
|---|---|---|---|---|
| N-[4-(2-{6-[4-diethylamino)styryl]-1-methyl pyridinium-2-yl}vinyl)phenyl]-N-ethyl ethanamine iodide BF-064 | 78.5 | 87 | 6830 | 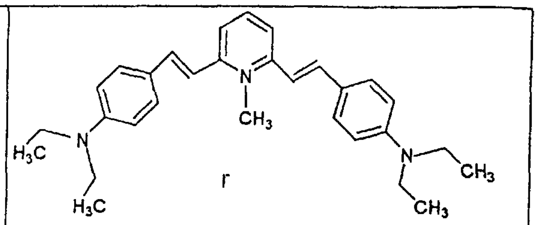 |
| 2-(4-(dimethyl amino)styryl)-1-ethyl pyridinium iodide SA-212 | 46.0 | | | 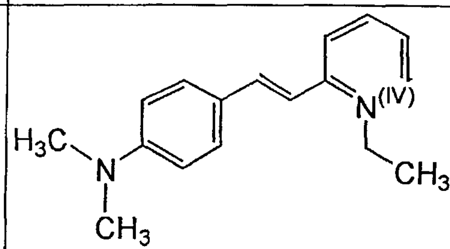 IH |
| 2-(4-(dimethyl amino)styryl)-1-methyl pyridinium iodide SA-213 | 50.7 | | | 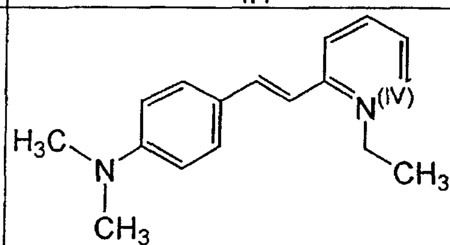 IH |
| 2-(4-(dimethyl amino)styryl) quinoline SA-770 | 25.4 | | | 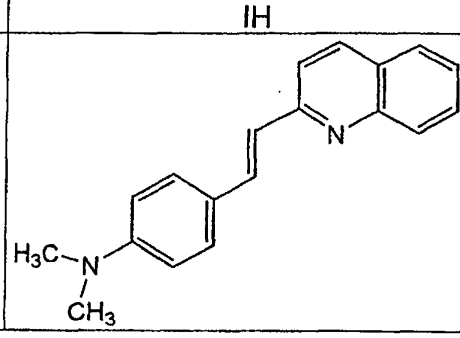 |

| | | | | |
|---|---|---|---|---|
| 2-(4-(dimetyl amino)stylyl)-1-methyl quinolinium iodide SA-245 | 86.9 | 10 | 869 | 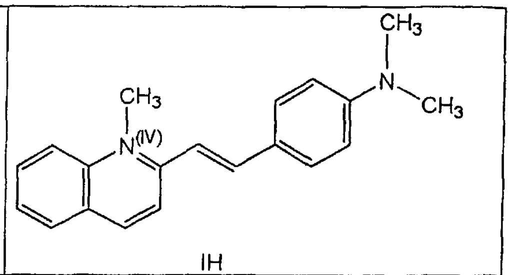 |
| Quinaldine Red SA-169 | 91.9 | 18 | 1654 | 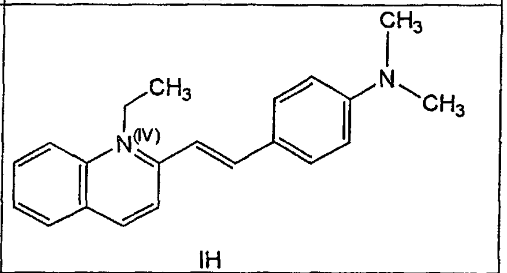 |
| 2-(4-(dimethyl amino)stylyl) pyridine SA-271 | 53.0 | | | 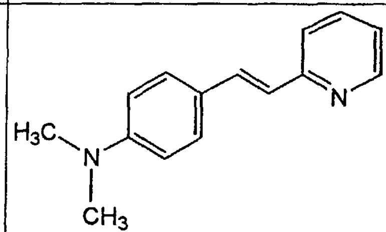 |
| 2-(4-(dimethyl amino)stylyl)-1-metyl pyridinium iodide SA-247 | 19.5 | | | 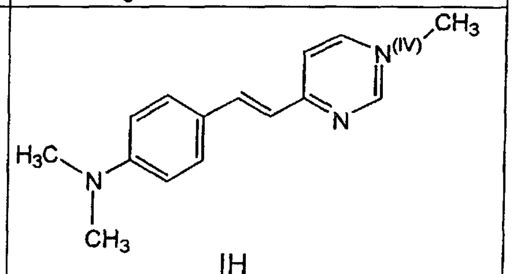 |
| 4-(4-diethylamino)stylyl)-1-metylpyridinium iodide SA-445 | 71.2 | | | 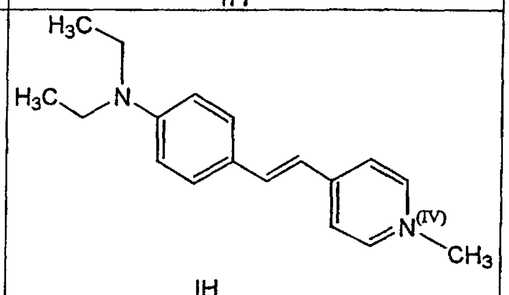 |
| 4,4'-diamino stylbene (2HCl salt) SA-324 | 36.0 | | | 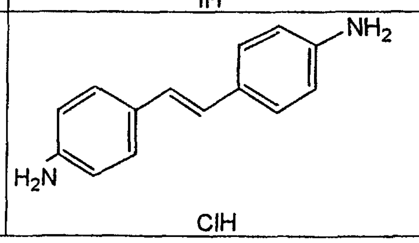 |

| | | | | |
|---|---|---|---|---|
| N-058 | 93.6 | | | 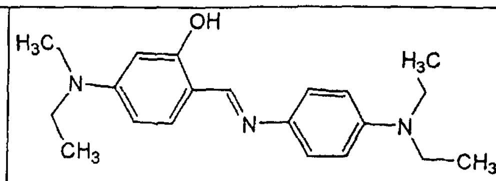 |
| BF-021 (N-058 HCl salt) | 70.8 | 30 | 2124 | 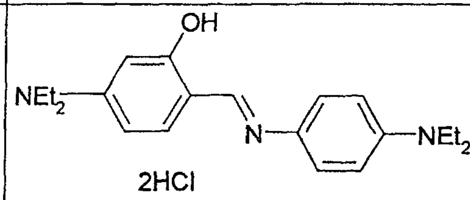 |
| N-(4-(dimethylamino)benzylidene)-p-phenetidine SA-399 | 23.1 | | | 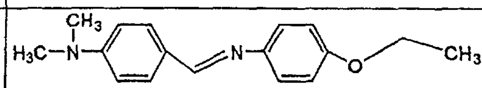 |
| BF-034 (SA-399 HCl salt) | 19.7 | | | 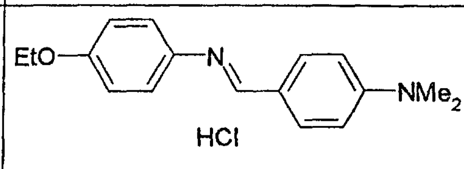 |
| BF-033 | 7.6 | | | 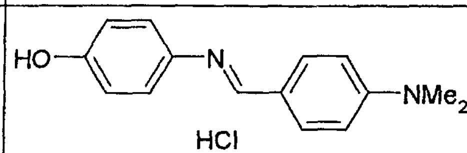 |
| N-(4-(dimethylamino)benzylidene)-4-fluoroaniline SA-407 | 6.8 | | | 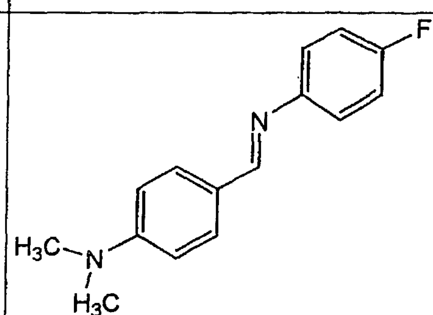 |
| BF-035 (SA-407 HCl salt) | 5.6 | | | 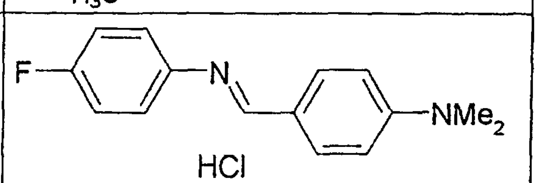 |

| | | | | |
|---|---|---|---|---|
| N-(4-(dimethylamino)benzylidene)-4-ethylaniline SA-408 | 7.9 | | | 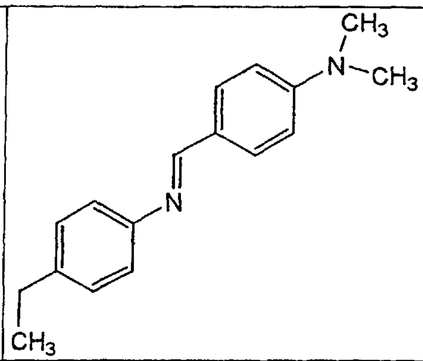 |
| N-(4-(diethylamino)benzylidene)aniline SA-757 | 33.9 | | | 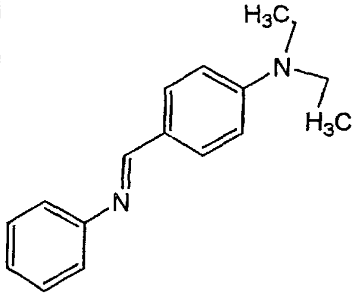 |
| N-benzylidene-N',N'-dimethylbenzene-1,4-diamine SA-758 | 46.5 | | | 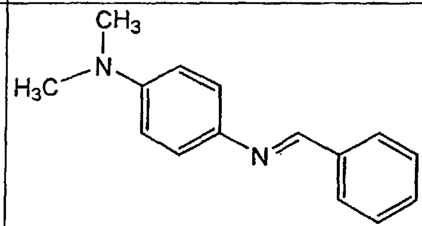 |
| benzo(F)isoquinoline-2-yl-(4-dimethylaminobenzylidene)-amine SA-672 | 39.3 | | | 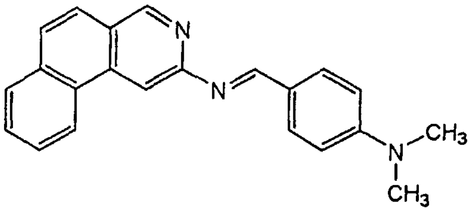 |
| 2-(5-bromo-2-yridylazo)-5-diethylamino)phenol SA-147 | 87.2 | | | 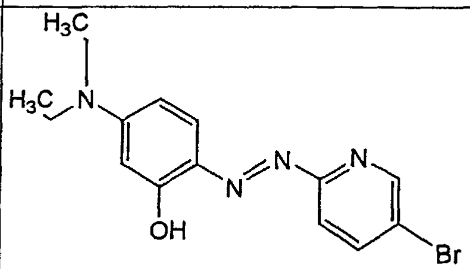 |

| | | | | |
|---|---|---|---|---|
| BF-020 (SA-147 HCl salt) | 84.4 | | | 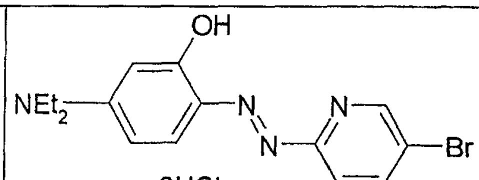 2HCl |
| Methyl Orange SA-125 | 62.2 | 1 | 62.2 | 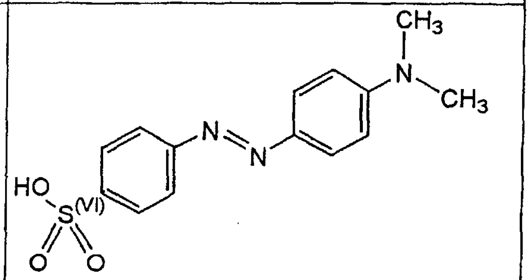 NaH |
| Ethyl Orange Na salt SA-163 | 30.7 | | | 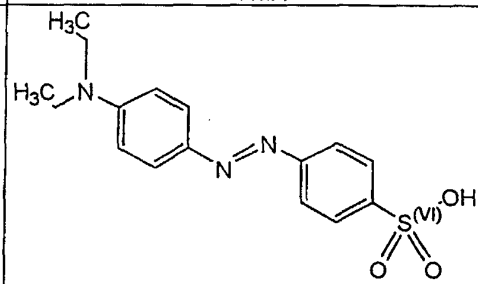 NaH |
| D5895 SA-606 | 53.5 | | | 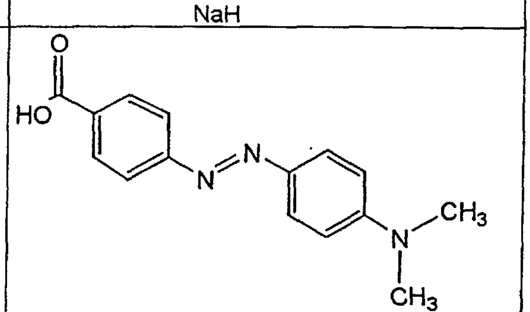 NaH |
| 4-(diethyl amino) azobenzen e SA-134 | 65.4 | | | 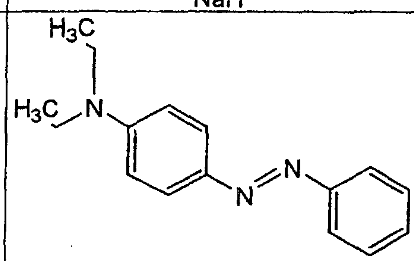 |
| 4-dimethyl amino-2-methyl-azobenzen e SA-135 | 42.8 | | | 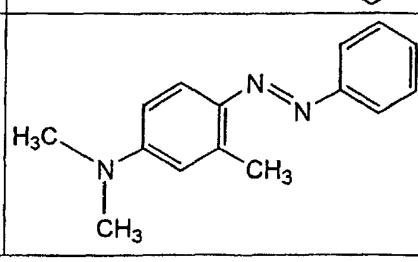 |

| | | | | |
|---|---|---|---|---|
| 4-phenyl azoaniline<br>HCl salt<br>SA-138 | 35.9 | | | 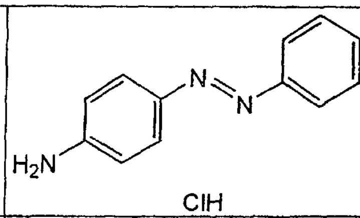 |
| 4-phenyl azoaniline<br>SA-148 | 28.0 | | | 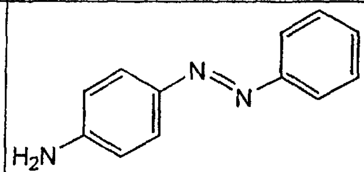 |
| Disprese Orange 3<br>SA-180 | 58.0 | | | 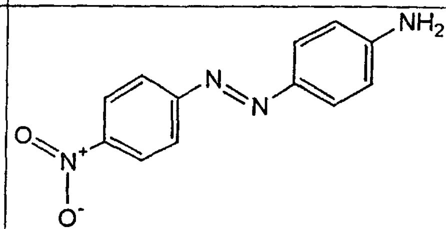 |
| N-(4-dimethyl amino) azobenzene-4'-iodo acetamide<br>SA-446 | 52.4 | | | 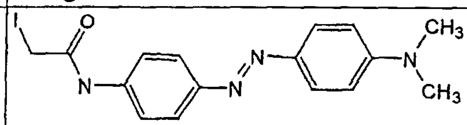 |
| 4-(dimethyl amino) azobenzene-4'-sulfonyl chloride<br>SA-188 | 62.7 | | | 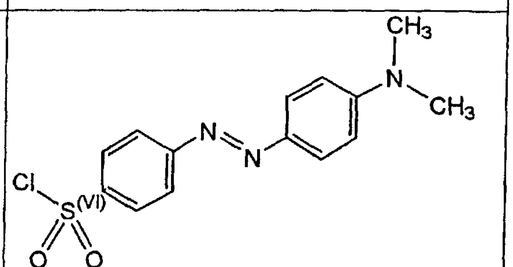 |
| Fat Brown RR<br>SA-201 | 73.0 | | | 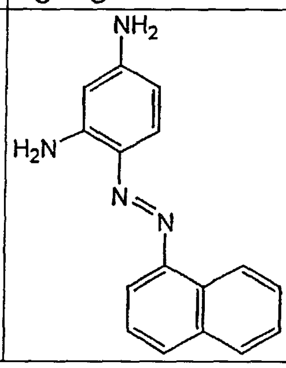 |

| | | | | |
|---|---|---|---|---|
| 4-(2-pyridylazo)-N,N-dimethyl aniline SA-215 | 69.1 | >100 | >6910 | 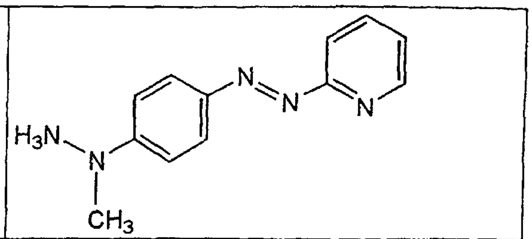 |
| 4-((4-isothiocyanatphenyl)azo)-N,N-dimethyl aniline SA-239 | 83.3 | | | 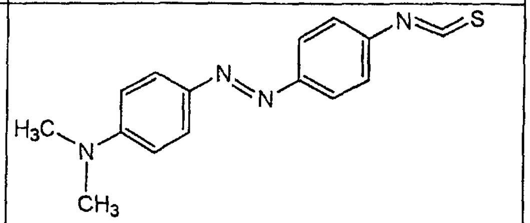 |
| N,N-dimethyl-4,4'-azodianiline SA-512 | 68.2 | | | 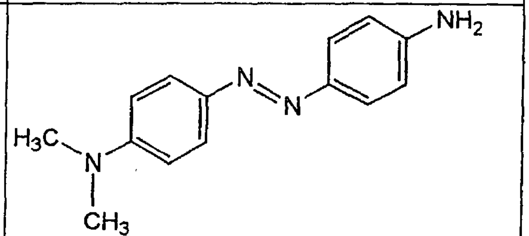 |
| 4,4' diamino-5-methoxy-2-methyl azobenzene SA-419 | 38.4 | | | 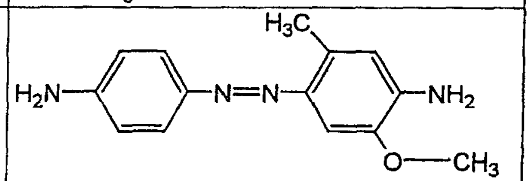 |
| 4-(diethyl amino)-4'-(dimethyl amino)-2-(ureido) azobenzene SA-420 | 71.6 | | | 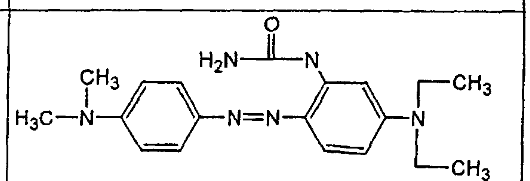 |
| BF-055 (SA-420 HCl salt) | 85.0 | | | 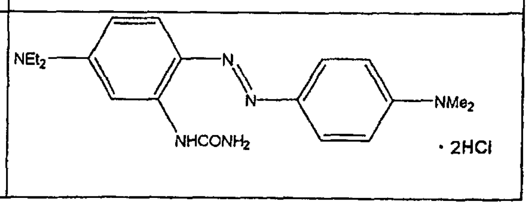 |

| | | | | |
|---|---|---|---|---|
| Disperse Black 7 SA-376 | 35.7 | | | 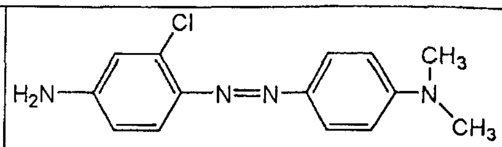 |
| Indophenol Blue SA-244 | 96.9 | | | 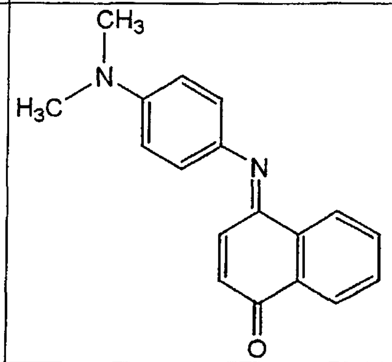 |
| BF-022 (SA-244 HCl salt) | 27.8 | | | 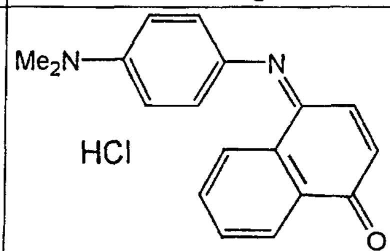 |
| 4-amino phenyl disulfide, SA-260 | 83.1 | | | 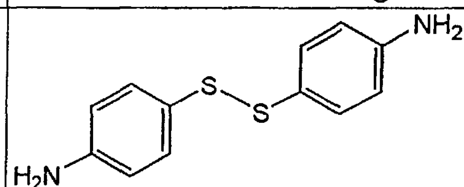 |
| N-200 | 21.6 | | | 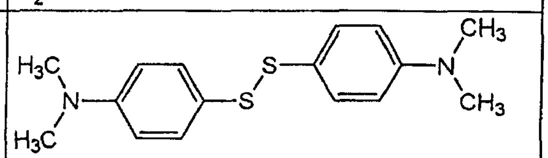 |
| 2,2'-dithiobis (1-naphthyl amine) SA-366 | 43.3 | | | 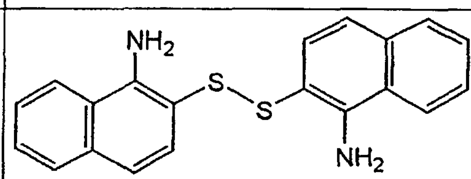 |
| Congo Red | 89.3 | 0.74 | 66 | 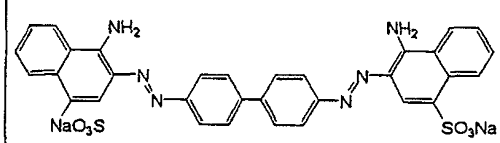 |

| | | | | |
|---|---|---|---|---|
| Chrysamine G | 66.8 | 9.7 (pH 5) | 649 | 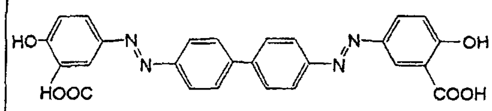 |
| Chrysamine G 2Na·$5H_2O$ | 84.5 | 2.7 | 228 | 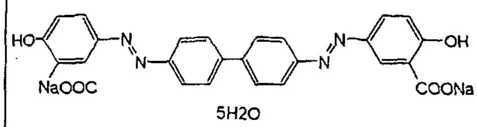 |
| X34 | 73.9 | 3.4 | 251 | 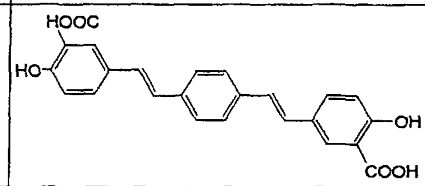 |
| X34·2Na | 74.0 | 2.7 | 200 | 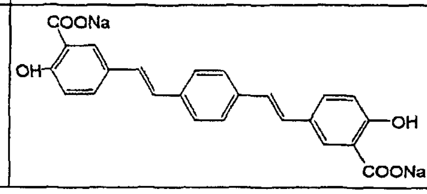 |

As shown in Table I, the compounds of the present invention belonging to a different group from that of the previously reported control compounds recognize the β structure. The β structure recognitions of the compounds of the present invention are on the same order as those of the control compounds. Among these compounds, N-039, BF-064, SA-245 ((dimethylaminostylyl)-1-methylquinoliniumiodide), SA-169 (Quinaldine red), N-058 (5-(diethylamino)-2-({[4-(diethylamino)phenyl]imino}methyl)phenol), SA-147 (2-(5-bromo-2-pyridylazo)-5-(diethylamino)phenol), SA-239 (4-((4-isothiocyanatphenyl)azo)-N,N-dimethylaniline), SA-420(4-(dietylamino)-4'-(dimethylamino)-2-(ureido)azobenzene, HCl salt), SA-244 (indophenol blue), and SA-260 (4-aminophenyldisulfide) highly recognize β structure. SA-215 also has relatively high recognition of β structure. The compounds of the present invention tend to have higher distribution coefficients than control compounds. SA-215 has the highest distribution coefficient, and N-039, BF-064 and BF-021 also have high distribution coefficients. Therefore, these compounds of the present invention have one or two order higher usefulness coefficients than control compounds. Compounds having particularly high usefulness coefficients are SA-215, BF-064 and N-039, the usefulness coefficient of which are >6910, 6830 and 3420, respectively. Usefulness coefficients of SA-169, BF-021 and SA-245 are also high. Therefore, it can be said from these results that the compounds of the present invention can specifically bind to the amyloid β protein and, moreover, have the extremely high blood-brain barrier permeability and, thus, have the extremely high value for utilizing as a probe for diagnosing diseases in which amyloid is accumulated.

Further, the insulin β structure recognitions of N-039, N-058, SA-147, SA-244, and SA-239 were measured by the method of the above (2), and the results are shown in Table II. As a control, β structure recognitions of Congo red and Chrysamine G were measured similarly. Measurement was carried out two times, and averages are shown in Table II. The insulin β sheet recognition of these compounds except SA-147 is similar to those of control, Congo red and Chrysamine G (see also Table I for compounds).

Table II

| Compound | Insulin β sheet structure recognition $EC_{50}$ (μM) |
|---|---|
| N-039 | 0.113 |
| N-058 | 0.129 |
| SA-147 | 0.038 |
| SA-244 | 0.449 |

Table II (continued)

| Compound | Insulin β sheet structure recognition $EC_{50}$ (μM) |
|---|---|
| SA-239 | 0.120 |
| Congo red | 0.153 |
| Chrysamine G | 0.253 |

As explained above, according to the present invention, there is provided a compound as a probe for imaging diagnosis of diseases in which amyloid is accumulated, which belong to a different group from that of the previous compound, has the high binding specificity to the amyloid β protein, and has the blood-brain barrier permeability. For that reason, according to the present invention, there are provided a compound as a probe for imaging diagnosis of diseases in which amyloid is accumulated, as well as a composition and a kit for imaging diagnosis of diseases in which amyloid is accumulated, which comprise the compound of the present invention. By using such a compound, composition or a kit, precise diagnosis can be done at an early stage of diseases becomes possible.

**Claims**

**1.** A compound used as a probe for imaging diagnosis of diseases in which amyloid is accumulated, represented by the formula I:

$$(R_1)_m—A—X\underline{\text{-----}}Y—B—(R_2)_n \qquad (I)$$

[wherein X represents CH, N or S;

Y represents CH, N or S; or absent;
$\underline{\text{----}}$ means a single bond or a double bond;
A and B each represents independently carbocyclic ring selected from benzene ring, naphthalene ring, anthracene ring and phenanthrene ring; or representssaid carbocyclic ring containing one to three heteroatoms selected from N, O and S;
$R_1$ represents a substituent for ring A, and each $R_1$ represents independently hydrogen, halogen, hydroxy, =O, alkyl having 1-4 carbons, -O-alkyl having 1-4 carbons, nitro, -N=S, amino, -O- alkyl having 1-4 carbons, mono-substituted amino with an alkyl having 1-4 carbons, di-substituted amino with alkyls having 1-4 carbons, $-NHCONH_2$, $-NHCOCH_2$-halogen, carboxyl, sulfonic acid group, $-SO_2$-halogen, $-SO_2-OH$, or

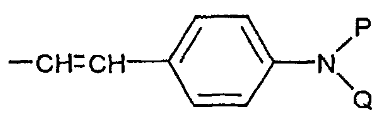

(wherein, P and Q each independently represents alkyl having 1-4 carbons);
$R_2$ represents a substituent for ring B; each $R_2$ represents independently hydrogen, halogen, hydroxy, =O, nitro, -N=S, amino, mono-substituted amino with an alkyl having 1-4 carbons, di-substituted amino with alkyls having 1-4 carbons, carboxyl, sulfonic acid group, or

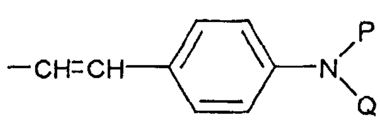

(wherein, P and Q independently are alkyl having 1-4 carbons);
n means any number of 1 to 7;
m means any number of 1 to 7;

provided -B- $(R_2)_n$ represents

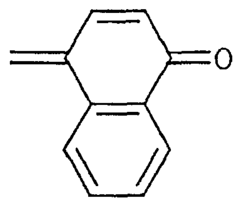

when Y means absent], or a salt or a solvate thereof.

**2.** A compound according to claim 1 selected from the group consisting of:

N-039 (N-[4-(2-{6-[4-(dimethylamino)styryl]-1-methylpiridinium-2-yl}vinyl)phenyl]-N-methylmethanamine), BF-064 (N-[4-(2-{6-[4-(dimethylamino)styryl]-1-methylpiridinium-2-yl}vinyl)phenyl]-N-ethylmethanamine) or SA-215 (4-(2-pyridylazo)-N,N-dimethylaniline),

or a salt or a solvate thereof.

**3.** The compound or a salt or a solvate thereof according to claim 1 or 2, which is labeled.

**4.** The compound or a salt or a solvated thereof according to claim 3, wherein the label is a radioactive nuclide.

**5.** The compound or a slat or a solvate thereof according to claim 4, wherein any of substituents $R_1$ to $R_2$ are labeled with a radioactive nuclide.

**6.** The compound or a salt or a solvate thereof according to claim 4, wherein hydrogen on the ring is substituted with a radioactive nuclide.

**7.** The compound or a salt or a solvate thereof according to any one claim of claims 4 to 6, wherein the label is a γ-ray-radiating nuclide.

**8.** The compound or a salt or a solvate thereof according to claim 7, wherein the γ-ray-radiating nuclide is selected from the group consisting of $^{99m}$Tc, $^{111}$In, $^{67}$Ga, $^{201}$Tl, $^{123}$I and $^{133}$Xe.

**9.** The compound or a salt or a solvate thereof according to claim 7, wherein the γ-ray-radiating nuclide is selected from the group consisting of $^{99m}$Tc and $^{123}$I.

**10.** The compound or a salt or a solvate thereof according to any one claim of 4 to 6, wherein the label is a positron-radiating nuclide.

**11.** The compound or a salt or a solvate thereof according to claim 10, wherein the positron-emitting nuclide is selected from the group consisting of $^{11}$C, $^{13}$N, $^{15}$O and $^{18}$F.

**12.** The compound or a salt or a solvate thereof according to claim 10, wherein a positron-emitting nuclide is $^{18}$F.

**13.** A composition for imaging-diagnosis of diseases in which amyloid is accumulated, which comprises the compound or a pharmaceutically acceptable salt or a solvate thereof described in any one claim of claims 1 to 12 and a pharmaceutically acceptable carrier.

**14.** The composition according to claim 13, which contains a compound selected from the group consisting of N-039, BF-064 and SA-205 labeled with $^{99m}$Tc or $^{123}$I or a pharmaceutically acceptable salt or a solvate thereof.

**15.** The composition according to claim 13, which contains a compound selected from the group consisting of N-039, BF-064 and SA-205 labeled with $^{18}$F or a pharmaceutically acceptable salt or solvate thereof.

**16.** A kit for imaging-diagnosis of diseases in which amyloid is accumulated, which comprises as an essential component the compound or a pharmaceutically acceptable salt or a solvate thereof described in any one claim of claims 1 to 12.

**17.** The kit according to claim 16, which comproses as an essential component a compound selected from the group consisting of N-039, BF-064 and SA-205 labeled with $^{99m\text{Tc}}$ or $^{123}$I or a pharmaceutically acceptable salt or a solvate thereof.

**18.** The kit according to claim 16, which comprises as an essential component a compound selected from the group consisting of N-039, BF-064 and SA-205 labeled with $^{18}$F or a pharmaceutically acceptable salt or a solvate thereof.

**19.** A use of the compound or a salt or a solvate thereof described in any one claim of claims 1 to 12, for preparing a composition or a kit for imaging-diagnosis of diseases in which amyloid is accumulated.

## INTERNATIONAL SEARCH REPORT

International application No.
PCT/JP01/02204

A. CLASSIFICATION OF SUBJECT MATTER
Int.Cl[7] C07C211/53, C07C251/24, C07C245/08, C07C309/46, C07C309/88, C07C331/28, C07C275/40, C07C251/22, C07C321/28, C07D213/38, C07D215/12, C07D221/10, C07D213/76, C07D239/26, A61K51/04 // C07M5:00, A61K100:10, A61K100:20, A61K100:02, A61K100:00, A61K123:00
According to International Patent Classification (IPC) or to both national classification and IPC

B. FIELDS SEARCHED
Minimum documentation searched (classification system followed by classification symbols)
Int.Cl[7] C07C211/53, C07C251/24, C07C245/08, C07C309/46, C07C309/88, C07C331/28, C07C275/40, C07C251/22, C07C321/28, C07D213/38, C07D215/12, C07D221/10, C07D213/76, C07D239/26, A61K51/04// C07M5:00, A61K100:10, A61K100:20, A61K100:02, A61K100:00, A61K123:00

Documentation searched other than minimum documentation to the extent that such documents are included in the fields searched

Electronic data base consulted during the international search (name of data base and, where practicable, search terms used)
CAPLUS(STN),REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | EP, 287909, A1 (SALUTAR, INC.), 26 October, 1988 (26.10.88), & JP, 63-290858, A & US, 4933156, A | 1-19 |
| X | WO, 98/40071, A1 (THE GENERAL HOSPITAL CORPORATION), 17 September, 1998 (17.09.98), & EP, 1007048, A1 | 1-19 |
| X | WO, 99/24394, A2 (UNIVERSITY OF PITTSBURGH), 20 May, 1999 (20.05.99), & EP, 1028941, A2 | 1-19 |
| X | EP, 290133, A2 (MINNESOTA MINING AND MANUFACTURING CO.), 09 November, 1988 (09.11.88), & JP, 63-273602, A & US, 5545676, A | 1-12 |
| X | ATTINA, Marina et al., "Labeled aryl fluorides from the nucleophilic displacement of activated nitro groups by fluoride-18 ion", J. Labelled Compd. Radiopharm., 1983, Vol.20, No.4, p.501-514 | 1-12 |

☒ Further documents are listed in the continuation of Box C.   ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 August, 2001 (14.08.01) | 14 August, 2001 (14.08.01) |
| Name and mailing address of the ISA/ Japanese Patent Office | Authorized officer |
| Facsimile No. | Telephone No. |

Form PCT/ISA/210 (second sheet) (July 1992)

INTERNATIONAL SEARCH REPORT

International application No.
PCT/JP01/02204

C (Continuation). DOCUMENTS CONSIDERED TO BE RELEVANT

| Category* | Citation of document, with indication, where appropriate, of the relevant passages | Relevant to claim No. |
|---|---|---|
| X | EP, 155551, A1(MITSUBISHI CHEMICAL INDUSTRIES LIMITED), 25 September, 1985 (25.09.85) & JP, 61-171440, A, esp., cis-stilbene, trans-stilbene | 1 |

Form PCT/ISA/210 (continuation of second sheet) (July 1992)